# EUROPEAN PATENT APPLICATION

(11) **EP 4 726 036 A1**
(43) Date of publication of application: **15.04.2026**
(21) Application number: 24864560.8
(22) Date of filing: 09.09.2024
(51) Int. Cl.: C12N 15/10, C40B 50/06, C12Q 1/6806

(54) **MRNA ENRICHMENT METHOD**

(30) Priority: 12.09.2023 CN 202311170349
(71) Applicant: Nanjing Vazyme Biotech Co., Ltd., Nanjing, Jiangsu 210046 (CN)
(72) Inventor: YI, Wenyang, Nanjing, Jiangsu 210046 (CN); ZHANG, Min, Nanjing, Jiangsu 210046 (CN); PAN, Yan, Nanjing, Jiangsu 210046 (CN); QU, Zhipeng, Nanjing, Jiangsu 210046 (CN); WANG, Tao, Nanjing, Jiangsu 210046 (CN)
(74) Representative: Pfenning, Meinig & Partner mbB
(86) International application number: PCT/CN2024/117636
(87) International publication number: WO 2025/055840

(57) **Abstract**

Provided is an mRNA enrichment method, relating to the technical field of biology. According to the method, first capture magnetic beads and second capture magnetic beads are respectively used for carrying out two rounds of capturing on mRNAs, such that rRNA residues in a product can be effectively reduced, and a high-purity mRNA product is obtained, thereby improving the data validity of downstream transcription analysis.

## Description

### Technical Field

The present application relates to the technical field of biology, and in particular to an mRNA enrichment method.

### Background Art

Over the past decade, transcriptome sequencing (RNA-seq) has become an important tool analyzing differential gene expression and differential splicing of mRNAs at a whole- transcriptome scale. The transcriptome refers to the collection of all transcription products in cells, tissues or organisms under a specific physiological condition, that is, the sum of all transcribed RNAs, including coding RNAs (mRNA) and non-coding RNAs (tRNA, rRNA, miRNA, lncRNA, circRNA), among other different types of RNA molecules. Among them, rRNA accounts for more than 80% of the total RNA, and is the most common type of RNA (Stark R, Grzelak M, Hadfield J. RNA sequencing: the teenage years. Nat Rev Genet. 2019 Nov;20(11):631-656.). As the most abundant member in total RNA, rRNA provides very little transcript information, and excessive detection will mask the expression abundance of other genes, thereby increasing the difficulty in detecting low-abundance transcripts. Therefore, rRNA is usually removed from the total RNA sample prior to sequencing, and the efficiency of rRNA removal is also regarded as a critical factor in maximizing reads mapping to transcripts.

Currently, most published RNA-seq data are based on Oligo(dT)-enriched mRNA. This enrichment method may select transcripts containing poly(A) tails and will focus the sequencing on the protein-coding regions in the transcriptome. However, the currently known mRNA capture protocols exhibit highly inconsistent performance in rRNA residue across different species. For example, they can achieve good enrichment effects on animal tissues, with the rRNA residue less than 5%, but the effect on different plants and fungi is very poor, where individual rRNA residues can be as high as 90%, which significantly limits the effectiveness of transcriptome analysis.

### Summary of the Invention

An objective of the present application is to provide an mRNA enrichment method capable of significantly reducing rRNA residues. The method is optimized on the basis of the existing technology. During the second time of mRNA enrichment, second capture magnetic beads are used for capturing, allowing mRNAs to bind to new capture magnetic beads, which can effectively reduce the rRNA residues in products and enhance the mRNA ratio, thereby facilitating the increased detection efficiency for low-abundance transcripts and the effectiveness of transcription analysis.

A first aspect of the present application provides an mRNA enrichment method comprising the steps of:
(1) mixing a total RNA sample with first capture magnetic beads, followed by incubation, wherein the first capture magnetic beads contain Oligo(dT) thereon;
(2) applying an external magnetic field to remove a supernatant;
(3) washing the first capture magnetic beads, and removing a wash buffer;
(4) eluting mRNAs on the first capture magnetic beads to obtain an eluted product;
(5) applying an external magnetic field, transferring the eluted product and discarding the first capture magnetic beads, and adding second capture magnetic beads to the eluted product followed by incubation, wherein the second capture magnetic beads contain Oligo(dT) thereon;
(6) applying an external magnetic field to remove a supernatant;
(7) washing the second capture magnetic beads, and removing a wash buffer; and
(8) eluting mRNAs on the second capture magnetic beads.

In some embodiments, the surfaces of the first and second capture magnetic beads are coupled to oligodeoxythymidylic acid chains (Oligo(dT)), which complementarily bind to the PolyA tail of the mRNA, wherein the number of nucleotides in the oligodeoxythymidylic acid chain is in a range of 5-50, preferably 5-30, including 5, 6, 7, 8, 9, 10, 11, 12, 13, 14, 15, 16, 17, 18, 19, 20, 21, 22, 23, 24, 25, 26, 27, 28, 29, and 30 within the range.

In some embodiments, the surface of each of the first capture magnetic beads and each of the second capture magnetic beads is coupled to at least one oligodeoxythymidylic acid chain (Oligo(dT)), for example at least 10, at least 100, at least 1,000, at least 5,000, at least 10,000, at least 20,000, at least 50,000, and at least 100,000 chains.

In some embodiments, the temperature for the incubation of step (1) is in a range of 50-80°C, preferably 55-75°C, including 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, and 75°C within the range.

In some embodiments, the time for the incubation of step (1) is in a range of 1-10 min, including 1 minute, 2 min, 3 min, 4 min, 5 min, 6 min, 7 min, 8 min, 9 min, and 10 min within the range.

In some embodiments, the condition for the incubation of step (1) is, for example incubation at 50-80°C for 1-10 min, for example incubation at 50-75°C for 1-10 min, for example incubation at 55-75°C for 1-10 min, for example incubation at 55-75°C for 1-8 min, for example incubation at 55-75°C for 2-8 min, for example incubation at 60-75°C for 2-8 min, for example incubation at 60-75°C for 3-8 min, for example incubation at 60-70°C for 3-8 min, for example incubation at 60-70°C for 3-7 min, for example incubation at 60-70°C for 3-6 min, for example incubation at 62-68°C for 3-6 min, for example incubation at 62-68°C for 4-6 min, and for example incubation at 65°C for 5 min, including but not limited to any combination of the aforementioned temperature for the incubation and the time for the incubation.

In some embodiments, the condition for the incubation of step (1) comprises incubation at 50-80°C for 1-10 min followed by incubation at room temperature for 1-10 min; preferably, incubation at room temperature for 1-10 min, for example 2-8 min, for example 2-7 min, for example 3-7 min, for example 4-6 min, and for example 5 min. The room temperature is in a range of 20-35°C, including 20°C, 21°C, 22°C, 23°C, 24°C, 25°C, 26°C, 27°C, 28°C, 29°C, 30°C, 31°C, 32°C, 33°C, 34°C, and 35°C within the range.

In some embodiments, the condition for the incubation of step (1) comprises incubation at 65°C for 5 min and incubation at room temperature for 5 min.

In some embodiments, the condition for the incubation of step (5) comprises incubation at room temperature for 1-10 min, for example 2-8 min, for example 2-7 min, for example 3-7 min, for example 4-6 min, and for example 5 min; and the room temperature is in a range of 20-35°C, including 20°C, 21°C, 22°C, 23°C, 24°C, 25°C, 26°C, 27°C, 28°C, 29°C, 30°C, 31°C, 32°C, 33°C, 34°C, and 35°C within the range.

In some embodiments, the condition for the incubation of step (5) comprises incubation at 50-80°C for 1-10 min followed by incubation at room temperature for 1-10 min; for example incubation at 60-70°C for 1-10 min followed by incubation at room temperature for 1-10 min; furthermore incubation at 60-70°C for 3-8 min followed by incubation at room temperature for 3-8 min; and furthermore incubation at 65°C for 5 min followed by incubation at room temperature for 5 min.

In some embodiments, the applying an external magnetic field of steps (2), (5) and (6) is, for example, placing the sample on a magnetic rack.

In some embodiments, the washing comprises adding a wash buffer, for example, adding a wash buffer to resuspend the magnetic beads, followed by pipetting and mixing homogeneously. In some embodiments, the wash buffer comprises, but is not limited to, all magnetic bead wash buffers known in the art, for example, the wash buffer contains salts and buffering ingredients. In some embodiments, the temperature for the washing is room temperature, for example in a range of 20-35°C, including 20°C, 21°C, 22°C, 23°C, 24°C, 25°C, 26°C, 27°C, 28°C, 29°C, 30°C, 31°C, 32°C, 33°C, 34°C, and 35°C within the range.

In some embodiments, the washing may be repeated one or two or more times.

In some embodiments, the eluting comprises adding an elution buffer including, but not limited to, all magnetic bead elution buffers known in the art, for example pure water or Tris buffer. The temperature for the eluting is 50-90°C, for example 50-85°C, for example 60-85°C, for example 70-85°C, for example 75-80°C, and for example 80°C. In some embodiments, the eluting comprises incubation for 1-10 min, for example 1-8 min, for example 1-6 min, for example 1-4 min, for example 1-3 min, and for example 2 min.

In some embodiments, the total RNA sample is derived from a eukaryote, including, but not limited to, animals, plants, protists, fungi, and the like.

In some embodiments, the animals include, but are not limited to, humans, mice, rats, guinea pigs, rabbits, dogs, monkeys, cattle, pigs, fish, turtles, cats, sheep, pigeons, chickens, frogs, toads, horses, cynomolgus monkeys, rhesus monkeys, orangutans, snakes, baboons, marmosets, cotton rats, ferrets, and the like; the fungi include, but are not limited to, yeast, rust fungi, *Geotrichum candidum, Streptomyces, Aspergillus flavus, Penicillium, Fusarium graminearum, Verticillium dahliae, Blumeria graminis, Aspergillus oryzae, Mycosphaerella graminicola, Aspergillus, Colletotrichum, Fusarium, Melampsora lini, Cryptococcus, Aspergillus fumigatus, Candida auris, Mucor, Candida albicans, Claviceps, Chaetomium,* mushrooms, etc., wherein the mushrooms are, for example, shiitake, straw mushroom, woodear, *Tremella fuciformis, Hericium erinaceus, Dictyophora indusiata, Agaricus bisporus, Tricholoma matsutake, Ganoderma lucidum, Cordyceps sinensis, Truffle, Boletus, Morchella esculenta,* and *Helvella elastica;* the protists include, but are not limited to, Paramecium, amoeba, Plasmodium, unicellular algae, slime molds, and the like; and the plants are, for example, seed plants or spore plants, including but not limited to, rice, *Arabidopsis thaliana,* tomato, wheat, camphor tree, carpet grass, raspberry, strawberry, apple, fir, pittosporum, linden, *Cnidium monnieri,* locust tree, sago cycas, tea tree, dandelion, soapberry, rhododendron, podocarpus, palm, ginkgo, garlic, pear, cherry, potato, areca palm, peach, *Forsythia,* osmanthus tree, longan, mango, oak, loquat, bamboo, barbary wolfberry, apricot tree, crabapple, persimmon tree, coconut tree, monstera, *Thaumatophyllum bipinnatifidum,* lychee, *Gluta spp.,* sugarcane, locust tree, China rose, elm, wisteria, *Betula platyphylla,* chrysanthemum, rapeseed, soybean, algae, fungi, lichen, bryophyte, and pteridophyte. The sampling part of the plant is, for example, a leaf, seed, root, flower, fruit, and stem.

A second aspect of the present application provides a method for constructing a transcriptome library, comprising the steps of:
(1) capturing mRNAs using the method of the first aspect;
(2) performing reverse transcription on the mRNAs to synthesize a first strand of cDNAs; optionally, synthesizing a second strand of cDNAs after the synthesis of the first strand of cDNAs;
(3) adding a transposase complex for incubation to obtain fragmented cDNAs;
(4) adding a primer with a adapter sequence to obtain an amplification product with the adapter sequence by PCR amplification; and
(5) optionally, purifying the amplification product.

In some embodiments, the reverse transcription uses M-MLV reverse transcriptase or AMV reverse transcriptase, preferably M-MLV reverse transcriptase.

In some embodiments, the primer used for the reverse transcription is an Oligo(dT) primer or a random hexamer, preferably a random hexamer.

In some embodiments, the transposase complex comprises a transposase and a transposon.

In some embodiments, the transposon comprises a transposase recognition core sequence, which is, for example, an OE sequence or an ME sequence.

In some embodiments, the transposon further comprises a sequencing primer sequence, which is single-stranded or double-stranded. The sequencing primer sequence can be of one or two types, for example, the read1 sequence and read2 sequence of the Illumina platform.

In some embodiments, the transposase binds to the transposon to form a transposase complex.

In some embodiments, two of the transposase complexes are conjugated and the ends interact to dimerize, forming a conjugated transposase complex consisting of one dimeric protein and two molecules of transposons, wherein the two molecules of transposons contain or do not contain a sequencing primer sequence. In some embodiments, the two molecules of transposons contain identical or different sequencing primer sequences.

In some embodiments, the amplification primer comprises a sequence that is identical or complementary to the transposase recognition core sequence.

In some embodiments, the amplification primer further comprises a sequence complementary to the sequencing primer sequence.

In some embodiments, the amplification primer further comprises an index sequence. In some embodiments, the index sequence contains, for example, 6-8 bases, and preferably 8 bases.

In some embodiments, the amplification primer is an amplification primer with a adapter sequence, wherein the amplification primer comprises a first primer with a first adapter sequence and a second primer with a second adapter sequence.

In some embodiments, the adapter sequence is a sequencing substrate-related sequence used by current or future sequencing platforms, including but not limited to the Ion Torrent platform, the Illumina platform, and the MGI platform.

In some embodiments, the first adapter sequence is a P5 adapter sequence of the Illumina platform, and the second adapter sequence is a P7 adapter sequence of the Illumina platform, or vice versa.

In some embodiments, the first adapter sequence is a P1 adapter sequence of the Ion Torrent platform, and the second adapter sequence is an A adapter of the Ion Torrent platform, or vice versa.

In some embodiments, the first adapter sequence is a single-end tag library construction upstream splint sequence of the MGI platform, and the second adapter sequence is a single-end tag library construction downstream splint sequence of the MGI platform, or vice versa. In some embodiments, the first adapter sequence is a paired-end tag library construction upstream splint sequence of the MGI platform, and the second adapter sequence is a paired-end tag library construction downstream splint sequence of the MGI platform, or vice versa.

In some embodiments, the first primer is an N5 primer, the second primer is an N7 primer, or vice versa.

In some embodiments, the transposase is a Tn5 transposase, which is any Tn5 transposase known in the art or an active mutant thereof.

In some embodiments, the Tn5 transposase is a Tn5 transposase having the activity of fragmenting RNA-cDNA hybrid strands.

In some embodiments, the method further comprises a step of terminating the fragmentation reaction, for example, by inactivating the transposase, such as by adding EDTA.

In some embodiments, the step of purifying employs a magnetic bead method, a high-salt precipitation method, a spin column method, or a phenol-chloroform extraction method, preferably a magnetic bead method.

A third aspect of the present application provides a method for constructing a transcriptome library, comprising the steps of:
(1) capturing mRNAs using the method of the first aspect;
(2) fragmenting the mRNAs to obtain fragmented mRNAs;
(3) performing reverse transcription on the fragmented mRNAs to synthesize cDNAs;
(4) performing end repair on the cDNAs;
(5) performing adapter ligation; and
(6) performing library amplification.

In some embodiments, the fragmenting comprises, but is not limited to, using an enzymatic fragmentation method, a chemical fragmentation method, and/or a mechanical fragmentation method to fragment the mRNAs. In some embodiments, the mechanical fragmentation method is, for example, an ultrasonic fragmentation method, a centrifugation method, or a heat shock method. The chemical fragmentation method is, for example, a treatment of the mRNAs with a chemical reagent to fragment them into multiple fragments. In some embodiments, the enzymatic fragmentation method is, for example, using one or more of Endonuclease V, Deoxyribonuclease I (Dnase I), and T7 endonuclease I.

In some embodiments, the reverse transcription uses M-MLV reverse transcriptase or AMV reverse transcriptase, preferably M-MLV reverse transcriptase. In some embodiments, the primer used for the reverse transcription is an Oligo(dT) primer or a random hexamer, preferably a random hexamer.

In some embodiments, the steps (2) and (3) may be combined into one step, or the steps (3) and (4) may be combined into one step, or the steps (4) and (5) may be combined into one step.

In some embodiments, the cDNA is a double-stranded cDNA.

In some embodiments, the end repair comprises performing end-filling on the cDNA to enable ligation of the cDNA to a blunt-end adapter.

In some embodiments, the end repair further comprises an A-tailing step, i.e. adding an adenine deoxyribonucleotide to the 3 'end of the cDNA after end-filling to achieve the ligation of the cDNA to the adapter via A-T.

In some embodiments, the enzyme for performing end-filling on the cDNA is, for example, T4 DNA polymerase. The enzyme used in the A-tailing step is, for example, Klenow DNA polymerase (3'-5' exo-).

In some embodiments, the adapter is a Y-shaped adapter of the Illumina platform, or a bubble-shaped adapter of the MGI platform, or a sequencing adapter of the Ion Torrent platform.

In some embodiments, T4 DNA ligase is used in the adapter ligation.

In some embodiments, the method further comprises purifying the product of the library amplification by using a magnetic bead method, a high-salt precipitation method, a spin column method, or a phenol-chloroform extraction method, preferably a magnetic bead method.

A fourth aspect of the present application provides a transcriptome analysis method comprising the steps of:
(1) constructing a library using the method of the second or third aspect;
(2) performing sequencing.

A fifth aspect of the present application provides a method for detecting mRNA expression, comprising:
(1) capturing mRNAs using the method of the first aspect;
(2) performing qRT-PCR detection.

A sixth aspect of the present application provides the use of capture magnetic beads in reducing rRNA residues, wherein the capture magnetic beads comprise first capture magnetic beads and second capture magnetic beads, and the first capture magnetic beads and the second capture magnetic beads have oligodeoxythymidylic acid (Oligo(dT)) chains coupled thereon.

A seventh aspect of the present application provides the use of the method of the first aspect in reducing rRNA residues.

### Brief Description of the Drawings

FIG. 1: Comparison of the operation procedures between the prior art (one-round capturing) and the technical solution of the present application (two-round capturing);
FIG. 2: The rRNA ratios detected by the two protocols when an input amount of the sample was 200 ng;
FIG. 3: The rRNA ratios detected by the two protocols when an input amount of the sample was 500 ng;
FIG. 4: The mRNA ratios detected by the two protocols when an input amount of the sample was 200 ng;
FIG. 5: The mRNA ratios detected by the two protocols when an input amount of the sample was 500 ng;
FIG. 6: The numbers of genes detected by the two protocols when an input amount of the sample was 200 ng;
FIG. 7: The numbers of genes detected by the two protocols when an input amount of the sample was 500 ng;
FIG. 8: The rRNA ratios detected by the two protocols when an input amount of the sample was 200 ng and mRNA Capture Beads from Yeasen were used;
FIG. 9: The rRNA ratios detected by the two protocols when an input amount of the sample was 500 ng and mRNA Capture Beads from Yeasen were used;
FIG. 10: The rRNA ratios detected by the two protocols for 11 different samples with an input amount of 500 ng;
FIG. 11: Optimized operation procedure of the two-round capturing technical solution;
FIG. 12: The rRNA ratios detected by using two two-round capturing protocols when an input amount of the sample was 500 ng.

### Detailed Description of Embodiments

The technical solutions of the present application will be further described below with reference to the accompanying drawings and through specific embodiments, but the following examples are merely simple examples of the present application, and do not represent or limit the scope of protection of the present application. The scope of protection of the present application shall be defined by the claims.

In the following examples, unless otherwise specified, the reagents and consumables used were purchased from conventional reagent manufacturers in the art. Unless otherwise specified, the experimental methods and technical means used were conventional methods and means in the art.

### Example 1

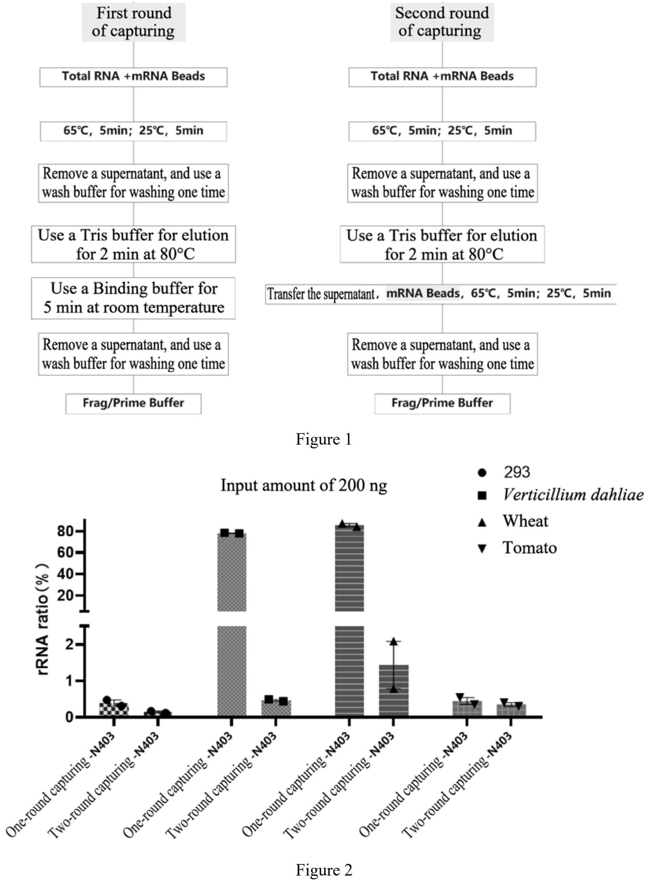

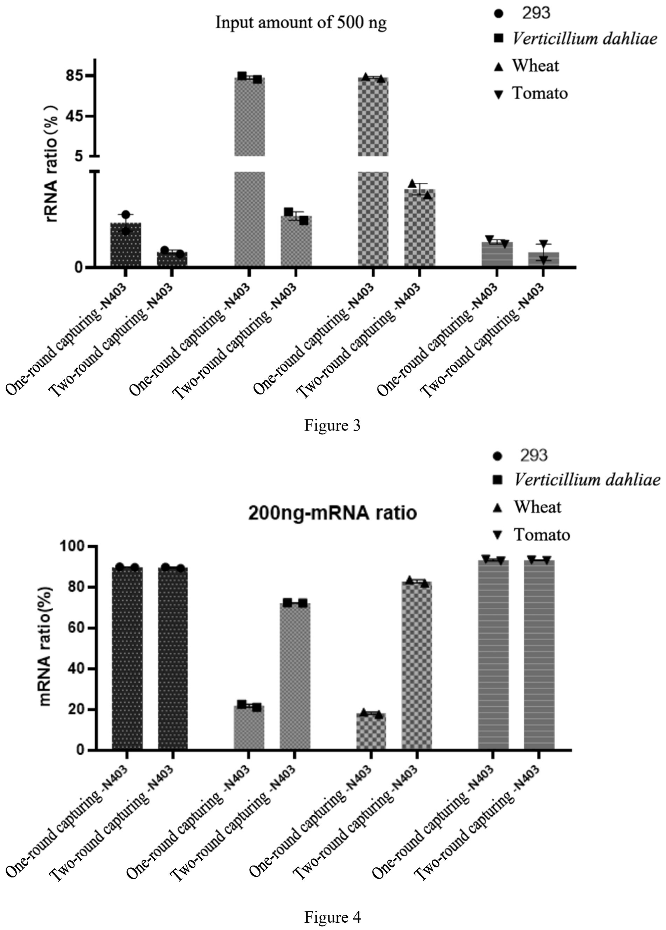

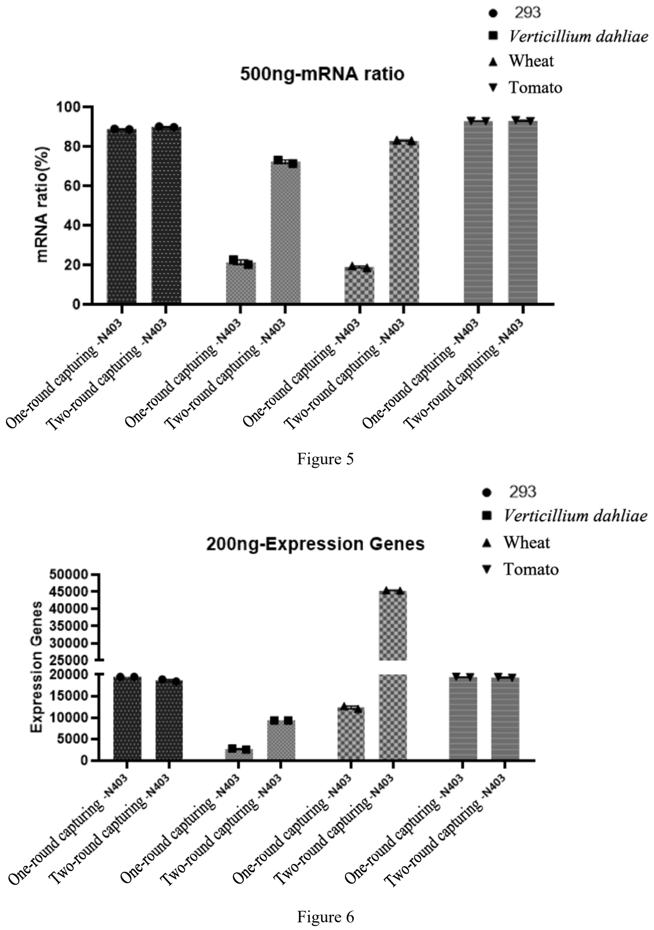

1. Total RNA extracted from 293 cells, tomato fruits, *Verticillium dahliae* and wheat seeds were used as samples, respectively. Protocol tests were carried out according to the procedures described in the instructions for Vazyme mRNA Capture Magnetic Beads Cat# N403. The beads of N403 contained Oligo(dT) thereon, which could capture mRNA through AT base complementarity. The input amounts of the total RNA were 200 ng and 500 ng, respectively. Two experimental protocols shown in FIG. 1 were adopted, respectively. The one-round capturing was a conventional method in the prior art (the flowchart on the left side of FIG. 1). In the one-round capturing, after the mRNA was eluted for the first time, a magnetic bead binding buffer was directly added to the eluted product for the magnetic beads to perform capturing on the mRNA for a second time (i.e., the beads used for the second time of capturing were the beads released from the previous step). The flowchart on the right side of FIG. 1 showed the two-round capturing method of the present application, and a step of transferring the supernatant and replacing old magnetic beads with new ones was added to the second time of mRNA capturing.
2. RNA-seq libraries were constructed from the above products, following the specific procedure described in the instructions for Vazyme #NR605.
3. The above constructed RNA-seq libraries were subjected to PE150 sequencing. The rRNA residues in the sequencing Reads were counted, and the results were as shown in FIGs. 2-3. The ratio of mRNA in the sequencing Reads was statistically analyzed, and the results were as shown in FIGs. 4-5. The number of genes detected in the sequencing results was statistically analyzed, and the results were as shown in FIGs. 6-7, where the Expression Gene in the ordinate referred to the number of genes to which mRNA reads were ultimately mapped.

The results showed that the innovative two-round capturing of the present application could significantly reduce rRNA residues. As shown in FIG. 3, with an input amount of the total RNA of 500 ng, the proportion of rRNA data decreased from 0.4% to 0.1% for 293 cells, from 83% to 0.5% for *Verticillium dahliae,* from 83% to 0.7% for wheat seeds, and from 0.2% to 0.1% for tomato fruits. Meanwhile, the methods provided by the present application were also conducive to increasing the ratio of mRNA and the number of genes detected. As shown in FIGs. 4 and 6, with an input amount of the total RNA of 200 ng, the ratio of mRNA data for the *Verticillium dahliae* sample increased from 21.5% to 72.3%, and the number of genes detected increased from 2,600 to 9,401.

### Example 2

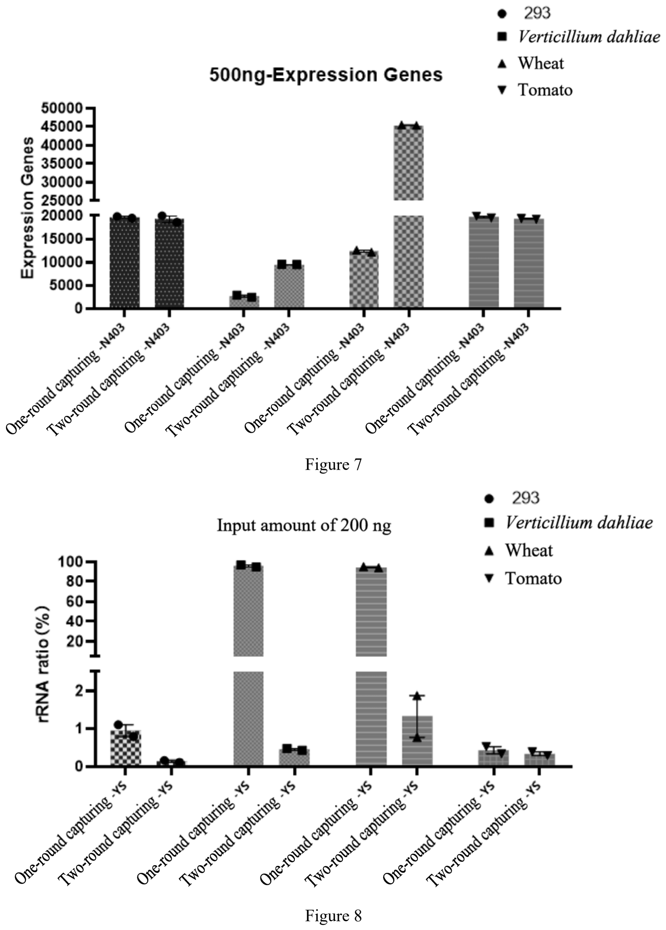

Tests were carried out by using mRNA Capture Beads from another company (Yeasen#12310ES96). Except for the magnetic beads, the sample types, input amounts and experimental procedures were the same as those in Example 1. The rRNA residues in the sequencing Reads were counted, and the results were as shown in FIGs. 8-9. As can be seen therefrom, with an input amount of the total RNA of 500 ng, the ratio of rRNA data decreased from 0.9% to 0.16% for 293 cells, from 96% to 0.5% for *Verticillium dahliae,* from 95% to 0.7% for wheat seeds, and from 0.5% to 0.1% for tomato fruits. The results showed that the methods provided by the present application were universally applicable to different magnetic beads, could reduce rRNA residues, and achieved the same effect as in that of Example 1.

### Example 3

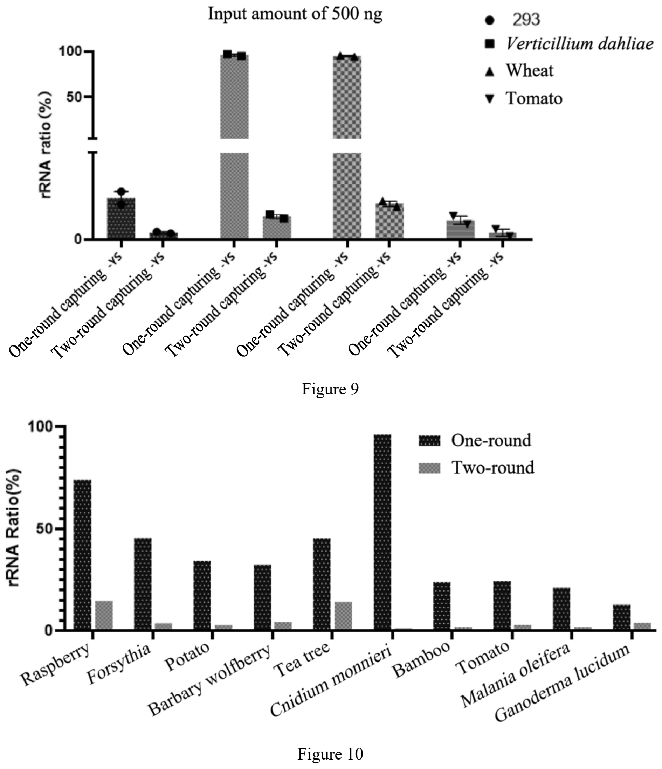

Tests were performed on the total RNA extracted from plant leaf samples (raspberry, *Forsythia,* potato, barbary wolfberry, tea tree, bamboo, tomato, and *Malania oleifera*), *Cnidium monnieri* root and stem samples, and *Ganoderma lucidum* root and stem samples with severe rRNA residues. The input amount of each sample was 500 ng. The experimental procedure was the same as that of Example 1. The rRNA residues in the sequencing Reads were counted, and the results were as shown in FIG. 10. The results showed that, compared with the one-round capturing protocol of the prior art, the two-round capturing protocol provided by the present application could reduce the rRNA residues in the aforementioned various plant samples by more than 50%.

### Example 4

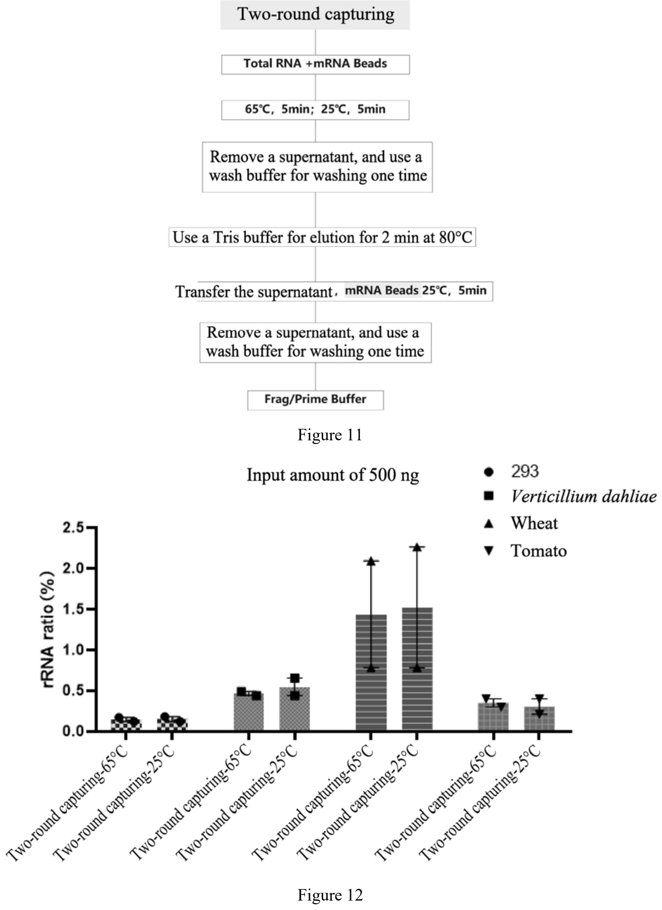

On the basis of Example 1, the two-round capturing technical solution was further optimized. In the step of the second time of mRNA capturing by magnetic beads, only an incubation treatment at 25°C for 5 min was set. The specific procedure was shown in FIG. 11,
wherein the samples and other experimental procedures were the same as those in the two-round capturing of Example 1. The input amount of each sample was 500 ng. The rRNA residues in the sequencing Reads were counted, and the results were as shown in FIG. 12. Comparing the data before and after optimization, the two treatment methods had little effects on rRNA residues, and the measured rRNA ratios were close, indicating that as long as new magnetic beads were used to complete the second round of capturing process, rRNA residues could be reduced.

## Claims

1. An mRNA enrichment method, comprising the steps of:
(1) mixing a total RNA sample with first capture magnetic beads, followed by incubation, wherein the first capture magnetic beads contain Oligo(dT) thereon;
(2) applying an external magnetic field to remove a supernatant;
(3) washing the first capture magnetic beads, and removing a wash buffer;
(4) eluting mRNAs on the first capture magnetic beads to obtain an eluted product;
(5) applying an external magnetic field, transferring the eluted product and discarding the first capture magnetic beads, and adding second capture magnetic beads to the eluted product followed by incubation, wherein the second capture magnetic beads contain Oligo(dT) thereon;
(6) applying an external magnetic field to remove a supernatant;
(7) washing the second capture magnetic beads, and removing a wash buffer; and
(8) eluting mRNAs on the second capture magnetic beads.

2. The method of claim 1, wherein the condition for the incubation of step (1) comprises incubation at 50-80°C for 1-10 min.

3. The method of claim 2, wherein the condition for the incubation of step (1) comprises incubation at 50-80°C for 1-10 min followed by incubation at room temperature for 1-10 min.

4. The method of claim 1, wherein the condition for the incubation of step (5) comprises incubation at room temperature for 1-10 min.

5. The method of claim 4, wherein the condition for the incubation of step (5) comprises incubation at 50-80°C for 1-10 min followed by incubation at room temperature for 1-10 min.

6. The method of claim 1, wherein the washing comprises adding the wash buffer, and the temperature for the washing is room temperature.

7. The method of claim 1, wherein the eluting comprises adding an elution buffer, and the condition for the eluting is incubation at 50-90°C for 1-10 min.

8. The method of claim 1, wherein the total RNA sample is derived from a eukaryote.

9. A method for constructing a transcriptome library, comprising the steps of:
(1) capturing mRNAs using the method of any one of claims 1-8;
(2) performing reverse transcription on the mRNAs to synthesize a first strand of cDNAs; optionally, synthesizing a second strand of cDNAs after the synthesis of the first strand of cDNAs;
(3) adding a transposase complex for incubation to obtain fragmented cDNAs;
(4) adding a primer with a adapter sequence to obtain an amplification product with the adapter sequence by PCR amplification; and
(5) optionally, purifying the amplification product.

10. The method of claim 9, wherein the transposase complex comprises a transposase, a transposase recognition core sequence, and a tag sequence.

11. The method of claim 9, wherein the primer further comprises an index sequence and/or a sequence complementary to the tag sequence.

12. The method of claim 9, wherein the primer comprises a first primer with a first adapter sequence and a second primer with a second adapter sequence.

13. The method of claim 12, wherein the first adapter sequence is a P5 adapter sequence of the Illumina platform, and the second adapter sequence is a P7 adapter sequence of the Illumina platform.

14. A method for constructing a transcriptome library, comprising the steps of:
(1) capturing mRNAs using the method of any one of claims 1-8;
(2) fragmenting the mRNAs to obtain fragmented mRNAs;
(3) performing reverse transcription on the fragmented mRNAs to synthesize cDNAs;
(4) performing end repair on the cDNAs;
(5) performing adapter ligation; and
(6) performing library amplification.

15. The method of claim 14, wherein the end repair comprises performing end-filling on the cDNAs.

16. The method of claim 15, wherein the end repair further comprises adding an adenine deoxyribonucleotide to the 3' end of the cDNAs.

17. The method of claim 14, wherein a method for the fragmenting is an enzymatic fragmentation method, a chemical fragmentation method, and/or a mechanical fragmentation method.

18. The method of claim 14, wherein the adapter is a Y-shaped adapter of the Illumina platform, or a bubble-shaped adapter of the MGI platform, or a sequencing adapter of the Ion Torrent platform.

19. A transcriptome analysis method, comprising the steps of:
(1) constructing a sequencing library using the method of any one of claims 9-18; and
(2) performing sequencing.

20. A method for detecting mRNA expression, comprising:
(1) capturing mRNAs using the method of any one of claims 1-8;
(2) performing qRT-PCR detection.
